# FASCICULE DE BREVET EUROPEEN

(11) **EP 1 743 015 B1**
(45) Date de publication et mention de la délivrance du brevet: **18.12.2013**
(21) Numéro de dépôt: 05769701.3
(22) Date de dépôt: 03.05.2005
(51) Int. Cl.: A61K 8/44, A61Q 5/02, A61Q 19/10, C07C 229/12

(54) **COMPOSITION TENSIOACTIVE, PROCEDE DE PREPARATION ET COSMETIQUE COMPRENANT CETTE COMPOSITION**
TENSIDZUSAMMENSETZUNG, VERFAHREN ZU IHRER HERSTELLUNG UND KOSMETIKPRODUKT DAMIT
SURFACTANT COMPOSITION METHOD FOR PRODUCTION THEREOF AND COSMETIC COMPRISING SAID COMPOSITION

(30) Priorité: 04.05.2004 FR 0404757
(43) Date de publication de la demande: 17.01.2007
(73) Titulaire: Eiffage Travaux Publics, 93330 Neuilly Sur Marne (FR); ECOLE NATIONALE SUPERIEURE DE CHIMIE DE RENNES, 35700 Rennes (FR)
(72) Inventeur: ANTOINE, Jean-Pierre, F-69340 Francheville (FR); MARCILLOUX, Jérôme, F-69360 Saint Symphorien d'Ozon (FR); LEFEUVRE, Martine, F-35235 Thorigne-Fouillard (FR); PLUSQUELLEC, Daniel, F-35230 Noyal Chatillon sur Seiche (FR); BENVEGNU, Thierry, F-35700 Rennes (FR); GOURSAUD, Fabrice, F-37320 Esvres sur Indre (FR); PARANT, Bernard, F-77330 Ozoir la Ferrière (FR)
(74) Mandataire: Bezault, Jean
(86) Numéro de dépôt international: PCT/FR2005/001103
(87) Numéro de publication internationale: WO 2005/121291

(56) Documents cités:
- EP-A- 0 750 904
- EP-A- 1 016 650
- DE-A- 3 527 974
- US-A- 2 888 383
- US-B1- 6 384 266
- DATABASE WPI Section Ch, Week 197445 Derwent Publications Ltd., London, GB; Class B05, AN 1974-78251V XP002312851 & JP 49 076818 A (AJINOMOTO KK) 24 juillet 1974 (1974-07-24)

## Description

L'invention concerne des compositions tensioactives.

Depuis la détergence jusqu'aux formulations cosmétiques, en passant par l'émulsification, la chimie des tensioactifs fournit une gamme extrêmement variée de produits aujourd'hui indispensables à notre vie quotidienne. Bien que le marché des amphiphiles cationiques soit quantitativement moins important que celui des anioniques ou des non ioniques, représentant, un pourcentage de la production mondiale inférieur à 10%, il est néanmoins très étendu et recouvre de multiples applications.

En raison de leur toxicité, certains tensioactifs tel que les sels de diméthyldialkylammonium, présents dans la plupart des adoucissants textiles, voient leur utilisation limitée, voire abandonnée, dans certains pays européens comme l'Allemagne et les Pays-Bas. Sous la pression de l'écologie, les producteurs de tensioactifs doivent proposer non seulement des procédés compatibles avec ces nouvelles exigences mais également des produits moins polluants, plus biodégradables et présentant une écotoxicité la plus faible possible. Aux contraintes environnementales s'ajoute un argument commercial de poids : le « naturel ». Pour répondre aux exigences du consommateur, soucieux de trouver des produits présentant une « image verte », les producteurs recherchent actuellement de nouvelles structures et se tournent naturellement vers l'utilisation de matières premières d'origine agricole.

Si, pour la partie lipophile de ces molécules, la concurrence est bien établie entre les matières pétrochimiques et les matières oléochimiques, elle n'est pas encore vraiment engagée en ce qui concerne la partie hydrophile de ces molécules. L'utilisation directe des triglycérides naturels, tels que le ricinoléate de triglycérol [US 4 857 310 (The Gillette Company)], permet d'accéder facilement à des amphiphiles cationiques. Mais la diversification des structures s'opère également par une modification de la tête polaire. Des tensioactifs cationiques, dérivés notamment d'acide glucuronique et d'acide galacturonique [DE 195 39 845 (Henkel KgaA)] ou à base d'amidon, comportant une entité sucre dérivée de polyglycosides d'alkyle (APG) [WO 90 15809 (Henkel KgaA)] ont été proposés. Un autre type de tensioactif apparaît. Il s'agit de molécules comprenant une fonction ester saponifiable entre la chaîne grasse et la fonction ammonium quaternaire, telles que les chlorures d'ammonium [US 5 527 477 (Lever Brothers Company)]. Facilement hydrolysables, les esters de bétaïnes suscitent aussi actuellement un vif intérêt. Divers dérivés ont été synthétisés ([WO 96 09276 (The Procter and Gamble Company)], [US 5 527 477 (Lever Brothers Company)]), visant des applications comme adoucissants textiles.

La glycine bétaïne, substance naturelle peu onéreuse, constitue une matière première de choix pour la préparation d'agents tensioactifs. Représentant 27% en poids de la mélasse de la betterave à sucre, obtenue après extraction du saccharose, elle reste actuellement un sous-produit de l'industrie sucrière. Le greffage sur la glycine bétaïne d'alcools et d'amines gras ([US 2 888 383 (International Minerals and Chemical Corporation)], [EP 0 750 904 A1 (Wella-AG)]) permet d'accéder à des molécules amphiphiles cationiques sans l'étape classique de quaternisation d'une amine tertiaire à l'aide d'agents de méthylation généralement toxiques.

La présente invention vise à proposer des voies d'accès rapides à des mélanges de composition parfaitement définie, à base d'esters ou d'amides de la glycine bétaïne, obtenus sous forme de bruts réactionnels ou par lavage des bruts réactionnels par des solvants organiques. Les procédés de synthèse mis en ouvre sont simples, efficaces, respectueux de l'environnement, sans solvant ni rejet polluant, facilement transposables à l'échelle industrielle et permettent la valorisation d'un sous-produit de l'industrie sucrière et des huiles végétales d'origine métropolitaine (par exemple colza ou tournesol), riches en chaînes grasses à condensation en carbone élevée (chaînes stéarylique, oléïque, linoléïque, linolénique, arachidique, gadolique, béhénique, érucique), encore peu valorisées par rapport aux ressources oléagineuses d'origine tropicale (par exemple palme, palmiste ou coprah), riches en chaînes caprylique, caprique, laurique, myristique ou palmitique.

Les chaînes grasses à 18 atomes de carbones issues des huiles végétales métropolitaines sont connues pour leurs propriétés émulsifiantes et les compositions selon l'invention couvrent une large gamme d'applications potentielles dans le domaine de l'émulsification, comme dans l'industrie du pétrole, dans l'industrie minière, dans l'industrie des peintures, pigments et vernis ou dans l'industrie du bâtiment et des travaux publics.

L'invention concerne également l'utilisation des mélanges mentionnés ci-dessus à titre d'agents détergents, émulsifiants ou moussants pour des applications dans le domaine cosmétique.

L'invention concerne notamment une composition tensioactive comprenant:
au moins un composé de formule (1)

   X⁻ (CH₃)₃N⁺-CH₂-CO-Z-R (1)

   et au moins un composé choisi parmi ceux des formules (2), (3) et (4)

   RZH (2)

   XH (3)

   X⁻ (CH₃)₃N⁺-CH₂-CO-OH (4)
X étant un radical sulfonate,
R étant un radical monovalent de formule C₂ₙH₂₍₂ₙ₋ₘ₎₊₁ contenant 2n atomes de carbone et m doubles liaisons, avec 9 ≤ n ≤ 11, 0 ≤ m ≤ 3 si n = 9 et 0 ≤ m ≤ 1 si n > 9, et
Z étant choisi parmi un atome d'oxygène et un groupe -NH-, un composé de formule XH étant combiné le cas échéant à au moins un composé de formule RNH₂ pour former au moins un composé de formule X⁻ RN⁺H₃.

Avantageusement, la composition selon l'invention comprend pratiquement exclusivement des composés desdites formules (1), (2), (3) et (4).

Des caractéristiques optionnelles de l'invention, complémentaires ou de substitution, sont énoncées ci-après:
- m = 0 et les composés constituant la composition sont les suivants, dans les pourcentages en poids indiqués:

| | |
|---|---|
| X⁻ (CH₃)₃N⁺-CH₂-CO-O-R | 50 ± 10 |
| ROH | 19 ± 10 |
| XH | 23 ± 10 |
| X⁻ (CH₃)₃N⁺-CH₂-CO-OH | 0 à 18. |

- m = 0 et les composés constituant la composition sont les suivants, dans les pourcentages en poids indiqués:

| | |
|---|---|
| X⁻ (CH₃)₃N⁺-CH₂-CO-O-R | 72 ± 10 |
| ROH | 0 à 20 |
| XH | 0 à 18 |
| X⁻ (CH₃)₃N⁺-CH₂-CO-OH | 0 à 20. |

- m = 0 et les composés constituant la composition sont les suivants, dans les pourcentages en poids indiqués:

| | |
|---|---|
| X⁻ (CH₃)₃N⁺-CH₂-CO-O-R | 80 ± 10 |
| ROH | 20 ± 10. |

- m = 0 et les composés constituant la composition sont les suivants, dans les pourcentages en poids indiqués:

| | |
|---|---|
| X⁻ (CH₃)₃N⁺-CH₂-CO-O-R | 70 ± 10 |
| ROH | 26 ± 10 |
| XH | 0 à 14. |

- m > 0 et les composés constituant la composition sont les suivants, dans les pourcentages en poids indiqués:

| | |
|---|---|
| X⁻ (CH₃)₃N⁺-CH₂-CO-O-R | 48 ± 10 |
| ROH | 36 ± 10 |
| XH | 14 ± 10 |
| X⁻ (CH₃)₃N⁺-CH₂-CO-OH | 0 à 12. |

- m = 0 et les composés constituant la composition sont les suivants, dans les pourcentages en poids indiqués:

| | |
|---|---|
| X⁻ (CH₃)₃N⁺-CH₂-CO-NH-R | 58 ± 10 |
| X⁻ RN⁺H₃ | 35 ± 10 |
| X⁻ (CH₃)₃N⁺-CH₂-CO-OH | 0 à 20. |

- m > 0 et les composés constituant la composition sont les suivants, dans les pourcentages en poids indiqués:

| | |
|---|---|
| X⁻ (CH₃)₃N⁺-CH₂-CO-NH-R | 56 ± 10 |
| X⁻ RN⁺H₃ | 31 ± 10 |
| RNH₂ | 0 à 18 |
| X⁻ (CH₃)₃N⁺-CH₂-CO-OH | 0 à 15. |

- X est choisi parmi les radicaux méthanesulfonate, paratoluènesulfonate et camphosulfonate.

L'invention a également pour objet un procédé de préparation d'une composition telle que définie ci-dessus, dans lequel on fait réagir la glycine bétaïne avec un acide sulfonique et un composé de formule ROH en l'absence d'autres solvants, le rapport molaire de l'acide sulfonique à la glycine bétaïne étant compris entre 2 et 3 et le rapport molaire du composé ROH à la glycine bétaïne étant compris entre 1 et 1,5.

Le procédé selon l'invention peut comporter au moins certaines des particularités suivantes:
- La réaction est conduite à une température comprise entre 130 et 140 °C pendant 6 à 8 heures..
- La réaction est conduite sous une pression réduite, de préférence comprise entre 5000 et 10000 Pa (50 et 100 mbar).
- m = 0 et on traite le mélange réactionnel par un solvant organique propre à dissoudre préférentiellement les composés autres que X⁻ (CH₃) ₃N⁺-CH₂-CO-O-R contenus dans celui-ci de manière à obtenir un précipité enrichi en X⁻ (CH₃)₃N⁺-CH₂-CO-O-R.
- Ledit solvant organique est choisi parmi l'éther diéthylique, l'éthanol et le n-butanol.
- On fait réagir la glycine bétaïne avec un acide sulfonique et le n-butanol en l'absence d'autres solvants, le rapport molaire de l'acide sulfonique à la glycine bétaïne étant compris entre 1 et 1,3 et le rapport molaire du n-butanol à la glycine bétaïne étant compris entre 2 et 4, pour former de l'eau et le sulfonate de l'ester n-butylique de glycine bétaïne, qu'on fait réagir, après avoir éliminé l'eau et le n-butanol, avec le composé de formule RNH₂, le rapport molaire de ce composé à la glycine bétaïne étant compris entre 1 et 1,2.
- La réaction de la glycine bétaïne avec l'acide sulfonique et le n-butanol est conduite à une température comprise entre 130 et 140°C, au reflux du n-butanol, pendant 3 à 5 heures sous pression atmosphérique de manière à réaliser l'élimination azéotropique de l'eau.
- On ajoute, avant le composé de formule RNH₂, une base organique forte et encombrée, notamment la dibutylamine, le rapport molaire de ladite base à la glycine bétaïne étant compris entre 0,1 et 0,4.
- m = 0 et on traite le mélange réactionnel par un solvant organique propre à dissoudre préférentiellement le composé RNH₂ de manière à obtenir un précipité enrichi en X⁻ (CH₃)₃N⁺-CH₂-CO-NH-R.
- Ledit solvant organique est l'éther diéthylique.
- Ledit acide sulfonique est choisi parmi l'acide méthanesulfonique, l'acide paratoluènesulfonique et l'acide camphosulfonique.

L'invention vise encore un cosmétique, en particulier savon liquide, bain moussant, gel douche ou shampoing et notamment shampoing acide, comprenant de 0,2 à 60 % et de préférence de 10 à 45 % en poids d'une composition telle que définie ci-dessus et de 99,8 à 40 % et de préférence de 90 à 55 % en poids d'excipients appropriés en cosmétologie.

Avantageusement, dans le cosmétique selon l'invention, les excipients sont choisis parmi des épaississants, des texturants, des agents de conditionnement, des adoucissants, des complexants, des parfums, des agents nacrants, des conservateurs, des acidifiants et de l'eau purifiée, et comprennent en tant que texturants des diéthanolamides d'acides gras, notamment le diéthanolamide de coprah, à une teneur ne dépassant pas 10 % en poids du cosmétique.

Un premier type de compositions selon l'invention est à base d'esters gras de la glycine bétaïne.

Le procédé de préparation de ces mélanges consiste à mettre en réaction la glycine bétaïne avec 2 à 3 équivalents molaires d'un acide sulfonique et 1 à 1,5 équivalent molaire d'un alcool gras saturé de type C_{18:0}, C_{20:0} ou C_{22:0} ou d'un alcool gras insaturé de type C_{18:1}, C_{18:2}, C_{18:3}, C_{20:1} ou C_{22:1}, les nombres situés avant et après le signe ":" représentant respectivement le nombre d'atomes de carbone et le nombre de doubles liaisons carbone-carbone. La réaction d'estérification de la glycine bétaïne présente sous forme zwitterionique nécessite une protonation préalable de sa fonction carboxylate. L'acide de la bétaïne réagit avec l'alcool gras en présence de l'excès d'acide pour conduire aux esters correspondants. Cette réaction s'effectue en l'absence de tout solvant, l'alcool gras utilisé constituant à la fois le réactif et le milieu.

De préférence, la réaction est mise en oeuvre à une température comprise entre 130 et 140 °C, pendant 6 à 8 heures. L'eau formée au cours de la réaction est éliminée en continu sous pression réduite, de préférence entre 5000 et 10000 Pa (50 et 100 mbar).

Les bruts réactionnels résultant de la mise en oeuvre du procédé décrit permettent d'accéder à des mélanges constitués d'ester gras de la glycine bétaïne, d'alcool gras résiduel, d'acide sulfonique résiduel et de glycine bétaïne résiduelle présente sous forme protonée, ces constituants étant affectés des coefficients a, b, c et d respectivement, dont les valeurs peuvent être déterminées par spectroscopie de RMN ¹H.

Lorsqu'un alcool gras saturé est utilisé dans la réaction, une purification rapide partielle basée sur la différence de solubilité entre l'alcool gras et l'ester synthétisé consiste en la reprise du brut réactionnel à l'aide de solvants organiques tels que l'éther diéthylique, l'éthanol ou le n-butanol en fin de réaction. L'ester, insoluble dans les solvants tels que l'éther diéthylique ou l'éthanol ou partiellement soluble, dans le butanol, précipite et l'alcool gras se solubilise partiellement. Par filtration, on obtient des poudres majoritairement constituées d'ester dont la composition massique peut être déterminée par spectroscopie de RMN ¹H.

Suivant le solvant organique utilisé pour le lavage des bruts réactionnels, les coefficients a, b, c et d varient. L'utilisation des alcools à chaînes carbonées courtes permet d'accéder à des mélanges où les coefficients c et d sont environ égaux à zéro, l'utilisation de l'éther diéthylique permet d'accéder à des mélanges présentant un taux d'alcool gras saturé résiduel (b) faible. La grande solubilité des alcools gras insaturés et de leurs esters correspondants dans les solvants organiques ne permet pas d'appliquer la méthode décrite et seuls sont accessibles les mélanges sous forme de bruts réactionnels.

On peut purifier les esters gras de la glycine bétaïne par chromatographie des mélanges selon l'invention sur colonne de gel de silice à l'aide d'éluants polaires ternaires de type acétate d'éthyle/isopropanol/eau.

Les esters gras de la glycine bétaïne présentent une fonction hydrolysable entre la chaîne grasse et l'ammonium quaternaire. L'étude de leur comportement en milieu aqueux permet d'apporter une information importante concernant leur biodégradabilité. La stabilité des dérivés est étudiée par chromatographie en phase gazeuse, en dosant l'alcool gras produit au cours de la réaction d'hydrolyse en solution aqueuse tampon dont le pH est fixé à diverses valeurs.

La mesure des tensions superficielles et des concentrations micellaires critiques prouvent que les dérivés synthétisés sont doués de propriétés amphiphiles qui permettent d'utiliser les mélanges proposés comme agents tensioactifs (notamment comme émulsifiants).

L'autre famille de mélanges selon l'invention est à base d'amide gras de la glycine bétaïne.

Le procédé de préparation de ces mélanges fait appel dans un premier temps à la réaction de la glycine bétaïne avec 1 à 1,3 équivalent molaire d'un acide sulfonique et 2 à 4 équivalents molaires de n-butanol constituant à la fois le réactif et le milieu, pour former l'ester n-butylique sous forme de sulfonate intermédiaire. De préférence, la première étape est réalisée à une température comprise entre 130 et 140 °C, au reflux du n-butanol, pendant 3 à 5 heures et sous pression atmosphérique. La distillation lente du n-butanol permet l'élimination azéotropique de l'eau formée au cours de la réaction. Au mélange refroidi à température ambiante est ensuite ajouté 1 à 1,2 équivalent molaire d'une amine grasse saturée de type C_{18:0}, C_{20:0} ou C_{22:0} ou d'une amine grasse insaturée de type C_{18:1}, C_{18:2}, C_{18:3}, C_{20:1} ou C_{22:1}. Le milieu réactionnel est chauffé sous pression réduite pour éliminer le n-butanol et l'aminolyse est menée pendant 2 à 4 heures, à 130 °C, entre 5000 et 10000 Pa (50 et 100 mbar).

La formation du sel d'amine grasse résultant de la protonation de l'amine par l'excès d'acide peut être limitée par l'utilisation de 0,1 à 0,4 équivalent molaire d'une base organique forte et encombrée telle que la dibutylamine ajoutée avant l'amine grasse.

Les bruts réactionnels résultant de la mise en oeuvre du procédé décrit permettent d'accéder à des mélanges constitués d'amide gras de la glycine bétaïne, du sulfonate de l'amine grasse utilisée lors de l'aminolyse, d'un faible résidu d'amine grasse et de glycine bétaïne résiduelle présente sous forme protonée, ces constituants étant affectés des coefficients e, f, g et h respectivement, dont les valeurs peuvent être déterminées par spectroscopie de RMN ¹H.

Lorsqu'une amine grasse saturée est utilisée dans la réaction, une purification rapide partielle basée sur la différence de solubilité entre l'amine grasse et l'amide synthétisé consiste en la reprise du brut réactionnel à l'aide d'éther diéthylique, en fin de réaction. L'amide et le sel de l'amine grasse insolubles dans le solvant précipitent et l'amine grasse se solubilise. Par filtration, on obtient des poudres majoritairement constituées d'amide dont la composition massique peut être déterminée par spectroscopie de RMN ¹H. L'utilisation de l'éther diéthylique permet d'accéder à des mélanges présentant un taux d'amine grasse saturée résiduelle (g) environ égal à zéro. La reprise des bruts réactionnels à l'aide d'alcools à chaînes carbonées courtes tels que l'éthanol ou le n-butanol entraîne la solubilisation des résidus.

La grande solubilité des amines grasses insaturés et des amides correspondants dans les solvants organiques ne permet pas d'appliquer la méthode décrite et seuls sont accessibles les mélanges sous forme de bruts réactionnels.

On peut purifier les amides gras de la glycine bétaïne par chromatographie des mélanges selon l'invention sur colonne de gel de silice à l'aide d'éluants polaires ternaires de type acétate d'éthyle/isopropanol/eau.

La mesure des tensions superficielles et des concentrations micellaires critiques prouve que les dérivés amides synthétisés sont également doués de propriétés amphiphiles qui permettent d'utiliser les mélanges proposés comme agents tensioactifs (notamment comme émulsifiants).

Les dérivés esters et amides de la glycine bétaïne présentent de bonnes propriétés tensioactives avec des tensions superficielles et des concentrations micellaires critiques relativement basses. Ces dérivés suivant l'invention présentent des tensions superficielles du même ordre de grandeur que les tensioactifs de référence comme les alkylpolyglucosides ou le dodécylsulfate de sodium (connu sous la marque déposée SDS), lequel est largement utilisé dans l'industrie. Par rapport à ces dérivés commerciaux, on obtient des concentrations micellaires critiques plus faibles, ce qui présente un intérêt majeur. Il faut en effet moins de produit pour obtenir des solutions micellaires.

En présence d'eau, ces dérivés s'hydratent plus ou moins bien selon la condensation en carbone des chaînes. Ils permettent ainsi d'homogénéiser un mélange eau-huile en agissant sur les interactions à la fois hydrophiles (tensioactif/eau) et lipophiles (tensioactif/huile). Ils permettent la formulation d'émulsions très stables, y compris pour les faibles concentrations en tensioactif, pour des rapports eau/tensioactif/huile variables et pour des huiles de différentes nature (en particulier les esters méthyliques d'acides gras).

Ces tensioactifs monocaténaires se révèlent très intéressants par leur pouvoir moussant et la stabilité des mousses formées puisqu'ils sont aussi performants que le SDS (marque déposée), d'où leur utilisation dans la formulation de produits moussants comme les shampoings, les savons liquides, les gels douche, les bains moussants.

Ainsi, l'invention fournit des cosmétiques comprenant de 0,2 à 60 % et de préférence de 10 à 45 % en poids d'une composition tensioactive selon l'invention et de 99,8 à 40% et de préférence de 90 à 55 % en poids d'excipients.

Un tel cosmétique peut être un savon liquide, un bain moussant, un gel douche ou un shampoing, notamment un shampoing acide doté d'un excellent pouvoir moussant, allié à un excellent pouvoir volumateur permettant de donner de la consistance à la coiffure. Un shampoing acide est par exemple composé de 10 à 45 % en poids d'une composition tensioactive selon l'invention à base d'esters gras de la glycine bétaïne et de 90 à 55 % d'adjuvants. Les adjuvants peuvent être des épaississants, des texturants, tels des diéthanolamide d'acides gras, notamment le diéthanolamide de coprah qui est incorporé à hauteur de 0-10 % en poids dans la formulation, des agents de conditionnement, des adoucissants, des complexants et finalement des parfums, des agents nacrants, des conservateurs, des acidifiants en quantité suffisante, et de l'eau purifiée.

L'invention est davantage illustrée par les exemples suivants.

### Exemple 1: Synthèse du mésylate de bétaïnate d'octadécyle et préparation des mélanges correspondants.

À une suspension de glycine bétaïne (25 g, 0,213 mole) dans l'acide méthanesulfonique (53,3 g, 0,555 mole) est ajouté de l'octadécanol (69,3 g, 0,256 mole). Le mélange ainsi obtenu est progressivement chauffé à 130 °C sous pression réduite (5000 à 6000 Pa) (50 à 60 mbars) pour éliminer l'eau formée au cours de la réaction d'estérification. Le mélange devient homogène au bout de 1 à 2 heures d'agitation à la même température. Après 7 heures, le milieu est refroidi à température ambiante.

Méthode A: Le brut réactionnel obtenu (143 g) constitue une première composition selon l'invention.

Méthode B: Le brut réactionnel obtenu est lavé à l'éther diéthylique (600 ml) jusqu'à la reprise complète du résidu. Le précipité obtenu est ensuite filtré sur büchner et rincé à plusieurs reprises avec le même solvant (2 × 200 ml). Le produit est séché sous vide et 96 g d'une poudre blanche sont finalement obtenus.

Méthode C: Le brut réactionnel obtenu est lavé à l'éthanol (800 ml) jusqu'à la reprise complète du résidu. Le précipité obtenu est ensuite filtré sur büchner et rincé à plusieurs reprises avec le même solvant (2 × 200 ml). Le produit est séché sous vide et 87 g d'une poudre blanche sont finalement obtenus.

Méthode D: Le brut réactionnel obtenu est lavé au n-butanol (900 ml) jusqu'à la reprise complète du résidu. Le précipité obtenu est ensuite filtré sur büchner et rincé à plusieurs reprises avec le même solvant (2 × 200 ml). Le produit est séché sous vide et 42 g d'une poudre légèrement teintée de gris sont finalement obtenus.

La composition de chaque mélange est évalué par RMN du proton en mesurant les rapports d'intégration entre les différents constituants. Les spectres RMN sont enregistrés sur un appareil Bruker ARX-400. Les spectres RMN ¹H sont obtenus à 400 MHz (s = singulet, d = doublet, t = triplet, m = multiplet, 1 = large). Les spectres RMN ¹³C sont obtenus à 100 MHz dans le mode découplage du proton. Les déplacements chimiques sont donnés en ppm par rapport à du TMS interne (échelle δ) et les constantes de couplage (J) en Hz.

RMN ¹H du brut réactionnel (CDCl₃+CD₃OD, 1/1) : δ 0,84 (t, CH₃ ester + CH₃ alcool, ³J 6,7 Hz), 1,22 (sl, (CH₂)₁₅CH₃ ester + (CH₂)₁₅CH₃ alcool), 1,51 (m, CH₂CH₂O alcool), 1,65 (m, CH₂CH₂O ester), 2,73 (s, CH₃SO₃⁻ ester + CH₃SO₃⁻ acide + CH₃SO₃⁻ glycine bétaïne), 3,29 (s, (CH₃)₃ ester + (CH₃)₃ glycine bétaïne), 3,51 (t, CH₂CH₂O alcool, ³J 6,7 Hz), 4,20 (t, CH₂CH₂O ester, ³J 6,8 Hz), 4,25 (s, CH₂CO glycine bétaïne), 4,33 (s, CH₂CO ester).

### Composition des mélanges

| Méthode de traitement | a | b | c | d |
|---|---|---|---|---|
| A | 50 | 19 | 23 | 8 |
| B | 72 | 10 | 8 | 10 |
| C | 80 | 20 | 0 | 0 |
| D | 70 | 26 | 4 | 0 |

| | | | | |
|---|---|---|---|---|
| a, b, c et d sont exprimés en pourcentage massique. | | | | |

Le brut réactionnel ou le produit issu de la méthode de traitement B est chromatographié sur colonne de gel de silice (acétate d'éthyle-isopropanol-eau (6,2:3:0,8)) pour donner environ 70 g de mésylate de bétaïnate d'octadécyle. 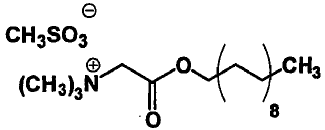 C₂₄H₅₁NO₅S; M = 465,74 g/mol
Solide blanc; rendement: ≅ 70%
CCM: Rf 0,39 (acétate d'éthyle-isopropanol-eau (6:3:1))
IR (nujol) ν (cm⁻¹): 1755 (C=O)
RMN ¹H (CDCl₃+CD₃OD, 1/1) :
8 0, 83 (t, 3H, CH₃, ³J 6,7 Hz), 1,22 (sl, 30H, (CH₂)₁₅CH₃), 1,65 (m, 2H, CH₂CH₂O), 2,70 (s, 3H, CH₃SO₃⁻), 3,29 (s, 9H, (CH₃)₃), 4,20 (t, 2H, CH₂CH₂O, ³J 6,7 Hz), 4,34 (s, 2H, CH₂CO).
RMN ¹³C (CDCl₃+CD₃OD, 1/1) :
δ 14, 33 (CH₃), 23, 17, 26,22, 28, 82, 29,71, 29,88, 30,02, 30,08, 30, 19, 32, 45 (CH₂ aliph.), 39,50 (CH₃SO₃⁻), 54,18 ((CH₃)₃), 63,46 (CH₂CH₂O), 67,27 (CH₂CO), 165,24 (CH₂CO) .

### Exemple 2: Synthèse du mésylate de bétaïnate de 9-octadécényle et préparation du mélange correspondant.

À une suspension de glycine bétaïne (30 g, 0,256 mole) dans l'acide méthanesulfonique (61,523 g, 0,64 mole) est ajouté de l'alcool oléïque (96,25 g, 0,359 mole). Le mélange ainsi obtenu est progressivement chauffé à 130 °C sous pression réduite 5000 à 10000 Pa (50 à 100 mbars) pour éliminer l'eau formée au cours de la réaction d'estérification. Le mélange devient homogène au bout de 1 à 2 heures d'agitation à la même température. Après 7 heures, le milieu est refroidi à température ambiante. Le brut réactionnel obtenu (210 g) constitue une composition selon l'invention.

RMN ¹H du brut réactionnel (CDCl₃):
δ 0,83 (t, CH₃ ester + CH₃ alcool, ³J 6,8 Hz), 1,22 (sl, CH₃(CH₂)₆CH₂CH=CHCH₂(CH₂)₅CH₂CH₂O ester + alcool), 1,50 (m, CH₂CH₂O alcool), 1,64 (m, CH₂CH₂O ester), 1,94 (m, CH₂CH=CHCH₂ ester + CH₂CH=CHCH₂ alcool), 2,74 (s, CH₃SO₃⁻ ester + CH₃SO₃⁻ acide + CH₃SO₃⁻ glycine bétaïne), 3,30 (s, (CH₃)₃ ester + (CH₃)₃ glycine bétaïne), 3,51 (t, CH₂CH₂O alcool, ³J 6,7 Hz), 4,19 (t, CH₂CH₂O ester, ³J 6,8 Hz), 4,24 (s, CH₂CO glycine bétaïne), 4,32 (s, CH₂CO ester), 5,30 (m, CH₂CH=CHCH₂ ester + CH₂CH=CHCH₂ alcool).

### Composition du mélange

| Méthode de traitement | a | b | c | d |
|---|---|---|---|---|
| A | 48 | 36 | 14 | 2 |

| | | | | |
|---|---|---|---|---|
| a, b, c et d sont exprimés en pourcentage massique. | | | | |

Le brut réactionnel est chromatographié sur colonne de gel de silice (acétate d'éthyle-isopropanol-eau (6,2:3:0,8 puis 6:3:1)) pour donner environ 100 g de mésylate de bétaïnate de 9-octadécényle. 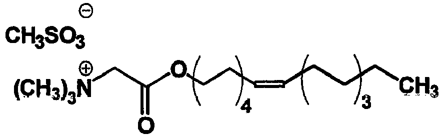 C₂₄H₄₉NO₅S; M=463,72 g/mol
Huile visqueuse jaune; rendement: ≅ 85%
CCM: Rf 0,4 (acétate d'éthyle-isopropanol-eau (6:3:1))
IR (nujol) ν (cm⁻¹): 1755 (C=O); 1650 (C=C)
RMN ¹H (CDCl₃) :
δ 0,83 (t, 3H, CH₃, ³J 6,8 Hz), 1,22 (sl, 22H, CH₃(CH₂)₆CH₂CH=CHCH₂(CH₂)₅CH₂CH₂O), 1,64 (m, 2H, CH₂CH₂O), 1,94 (m, 4H, CH₂CH=CHCH₂), 2,71 (s, 3H, CH₃SO₃⁻), 3,30 (s, 9H, (CH₃)₃), 4,19 (t, 2H, CH₂CH₂O, ³J 6, 8 Hz), 4, 32 (s, 2H, CH₂CO), 5,31 (m, 2H, CH₂CH=CHCH₂).
RMN ¹³C (CDCl₃) :
δ 14,21 (CH₃), 22,90, 25,89, 27,40, 28,49, 29,38, 29,52, 29,62, 29,73, 29,86, 29,91, 29,96, 32,13, 32,81 (CH₂ aliph.), 39,27 (CH₃SO₃⁻), 54,09 ((CH₃)₃), 63,22 (CH₂CH₂O), 67,09 (CH₂CO), 129,93, 130,24 (CH₂CH=CHCH₂), 164,88 (CH₂CO).

### Exemple 3: Synthèse du mésylate de bétaïnylaminooctadécane et préparation d'un mélange correspondant.

Une suspension de glycine bétaïne (25 g, 0,213 mole) dans le n-butanol (59 ml, 0,64 mole) est réalisée en présence d'acide méthanesulfonique (22,56 g, 0,235 mole). Le mélange réactionnel est porté au reflux du n-butanol à 140 °C. Le milieu devient homogène au bout de 3 à 4 heures d'agitation. Au mélange refroidi à température ambiante est ajoutée de la dibutylamine (8,27 g, 0,064 mole) et le milieu est agité 15 minutes environ. De l'octadécylamine est ensuite ajoutée (69 g, 0,256 mole), puis le n-butanol est éliminé sous pression réduite. L'aminolyse est menée à 130 °C sous pression réduite (5000 à 10000 Pa) (50 à 100 mbars). Après 3 heures, le milieu est refroidi à température ambiante. Le brut réactionnel obtenu est lavé à l'éther diéthylique (1600 ml) jusqu'à la reprise complète du résidu. Le précipité obtenu est ensuite filtré sur büchner et rincé à plusieurs reprises avec le même solvant (2 × 200 ml). Le produit est séché sous vide et 98 g d'une poudre blanche d'une composition tensioactive selon l'invention sont finalement obtenus.

RMN ¹H du mélange obtenu (CDCl₃+CD₃OD, 1/1) :
δ 0,90 (t, CH₃ amide + CH₃ sel d'aminé, ³J 6,7 Hz), 1,29 (sl, (CH₂)₁₅CH₃ amide + (CH₂)₁₅CH₃ sel d'amine), 1,56 (m, CH₂CH₂NH amide), 1,67 (m, CH₂CH₂NH₃⁺ sel d'amine), 2,78 (s, CH₃SO₃⁻ amide + CH₃SO₃⁻ sel d' amine + CH₃SO₃⁻ glycine bétaïne), 2,91 (m, CH₂CH₂NH₃⁺ sel d'amine), 3,26 (m, CH₂CH₂NH amide), 3,30 (s, (CH₃)₃ glycine bétaïne), 3,35 (s, (CH₃)₃ amide), 3,82 (s, CH₂CO glycine bétaïne), 4,10 (s, CH₂CO amide).

### Composition du mélange

| Méthode de traitement | e | f | g | h |
|---|---|---|---|---|
| B | 58 | 35 | 0 | 10 |

| | | | | |
|---|---|---|---|---|
| e, f, g et h sont exprimés en pourcentage massique du mélange | | | | |

Le produit issu de la méthode de traitement B est chromatographié sur colonne de gel de silice (acétate d'éthyle-isopropanol-eau (6:3:1 puis 5:3:2)) pour donner environ 58 g de mésylate de bétaïnylaminooctadécane. 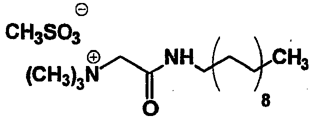 C₂₄H₅₂N₂O₄S; M=464, 75 g/mol
Solide blanc; rendement: ≅ 60%
CCM: Rf 0,5 (acétate d'éthyle-isopropanol-eau (5:3:2))
IR (nujol) ν (cm⁻¹): 1680 (Amide I); 1578 (Amide II)
RMN ¹H (DMSO):
δ 0,86 (t, 3H, CH₃, ³J 6, 8 Hz), 1,25 (sI, 30H, (CH₂)₁₅CH₃), 1,45 (m, 2H, CH₂CH₂NH), 2,37 (s, 3H, CH₃SO₃⁻), 3,10 (m, 2H, CH₂CH₂NH), 3,21 (s, 9H, (CH₃)₃), 4,05 (s, 2H, CH₂CO), 8,64 (s, 1H, NH).
RMN ¹³C (DMSO) :
δ 13,40 (CH₃), 21,63, 24,22, 26,01, 28,27, 28, 59, 30,88 (CH₂ aliph.), 38,50 (CH₂NH), 39,42 (CH₃SO₃⁻), 53,35 ((CH₃)₃), 64, 24 (CH₂CO), 162,66 (CH₂CO).

### Exemple 4: Synthèse du mésylate de bétaïnylaminooctadécényle et préparation du mélange correspondant.

Une suspension de glycine bétaïne (25 g, 0,213 mole) dans le n-butanol (59 ml, 0,64 mole) est réalisée en présence d'acide méthanesulfonique (22,56 g, 0,235 mole). Le mélange réactionnel est porté au reflux du n-butanol à 140 °C. Le milieu devisent homogène au bout de 3 à 4 heures d'agitation. Au.mélange refroidi à température ambiante est ajoutée de l'amine oléïque (68,5 g, 0,256 mole), puis le n-butanol est éliminé sous pression réduite. L'aminolyse est menée à 130-140 °C sous pression réduite (5000 à 10000 Pa) (50 à 100 mbars). Après 3 heures, le milieu est refroidi à température ambiante. Le brut réactionnel obtenu (114 g) constitue une composition tensioactive selon l'invention.
RMN ¹H du brut réactionnel (CDCl₃) :
δ 0,86 (t, CH₃ amide + CH₃ sel d'amine + CH₃ amine, ³J 6,7 Hz), 1,29 (sl, CH₃(CH₂)₆CH₂CH=CHCH₂(CH₂)₅CH₂CH₂N amide + sel d'amine + amine + CH₂CH₂NH₂ amine), 1,52 (m, CH₂CH₂NH amide), 1,61 (m, CH₂CH₂NH₃⁺ sel d'amine), 1,98 (m, CH₂CH=CHCH₂ amide + sel d'amine + amine), 2,67 (t, CH₂CH₂NH₂ amine, ³J 6,8 Hz), 2,73 (s, CH₃SO₃⁻ amide + CH₃SO₃⁻ sel d'amine + CH₃SO₃glycine bétaïne), 2,87 (m, CH₂CH₂NH₃⁺ sel d'amine), 3,21 (m, CH₂CH₂NH amide), 3,27 (s, (CH₃)₃ glycine bétaïne), 3,31 (s, (CH₃)₃ amide), 3,77 (s, CH₂CO glycine bétaïne), 4,08 (s, CH₂CO amide), 5,30 (m, CH₂CH=CHCH₂ amide + sel d'amine + amine).

### Composition du mélange

| Méthode de traitement | e | f | g | h |
|---|---|---|---|---|
| A | 56 | 31 | 8 | 5 |

| | | | | |
|---|---|---|---|---|
| e, f, g et h sont exprimés en pourcentage massique du mélange. | | | | |

Le brut réactionnel est chromatographié sur colonne de gel de silice (acétate d'éthyle-isopropanol-eau (6:3:1 puis 5:3:2)) pour donner environ 64 g de mésylate de bétaïnylaminooctadécényle. 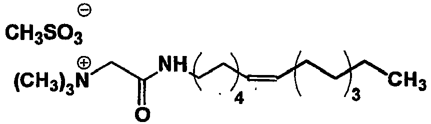 C₂₄H₅₀N₂O₄S; M=462,74 g/mol
Solide amorphe blanc; rendement: ≅ 65%
CCM: Rf 0,52 (acétate d'éthyle-isopropanol-eau (5:3:2))
IR (nujol) ν (cm⁻¹): 1678 (Amide I) ; 1576 (Amide II); 1640 (C=C)
RMN ¹H (DMSO):
δ 0,84 (t, 3H, CH₃, ³J 6,7 Hz), 1,23 (sl, 22H, CH3(CH₂)₆CH₂CH=CHCH₂(CH₂)₅CH₂CH₂NH), 1,40 (m, 2H, CH₂CH₂NH), 1,97 (m, 4H, CH₂CH=CHCH₂), 2,33 (s, 3H, CH₃SO₃⁻), 3,08 (m, 2H, CH₂CH₂NH), 3,20 (s, 9H, (CH₃)₃), 4,07 (s, 2H, CH₂CO), 5,31 (m, 2H, CH₂CH=CHCH₂), 8,61 (s, 1H, NH).
RMN ¹³C (DMSO):
δ 14,08 (CH₃), 22,20, 26, 43, 26, 65, 26,69, 28, 69, 28,73, 28,80, 28, 92, 28,96, 29,17, 29,22, 31,38, 32,04 (CH₂ aliph.), 38,66 (CH₂NH), 39,78 (CH₃SO₃⁻), 53,25 ((CH₃)₃), 63,86 (CH₂CO), 129,74, 129,75 (CH₂CH=CHCH₂), 163,19 (CH₂CO).

On donne ci-après les propriétés physico-chimiques de certains dérivés selon l'invention.

### Mesure des tensions superficielles et des concentrations micellaires critiques

Les mesures tensiométriques ont été effectuées à l'aide d'un tensiomètre à goutte selon la méthode de la goutte montante (tensiomètre TRACKER, I.T. CONCEPT).

| Composés amphiphiles | γ_{cmc} (mN/m) | CMC (M) |
|---|---|---|
| Esters | | |
| C_{18:0} | 37,2 | 8.10⁻⁴ |
| C_{18:1} | 36,6 | 1.10⁻⁴ |

| Amides | | |
|---|---|---|
| C_{18:0} | 36,0 | 1,4.10⁻⁴ |
| C_{18:1} | 36,2 | 1,2.10⁻⁴ |

| Exemples de tensioactifs très utilisés | | |
|---|---|---|
| APG (C₁₂) | 33,0 | 2,6.10⁻⁴ |
| SDS (C₁₂) | 30,0 | 8.10⁻³ |

### Stabilité des esters gras de la glycine bétaïne en milieu aqueux

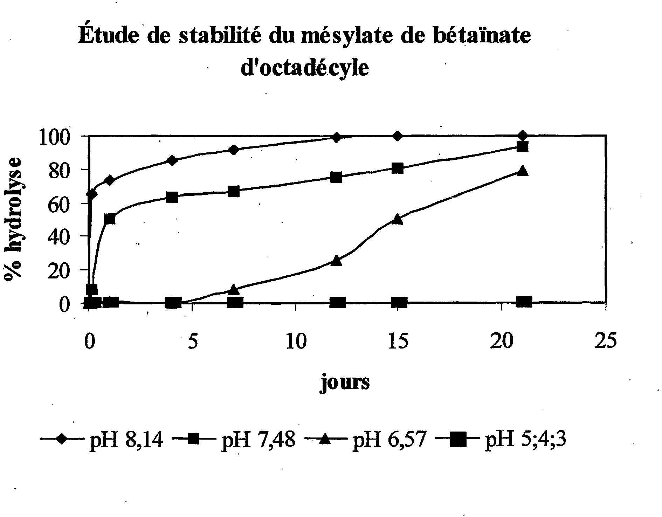

La stabilité du mésylate de bétaïnate d'octadécyle est étudiée en solution aqueuse tampon dont le pH est fixé à diverses valeurs comprises entre 3 et 9. La concentration initiale en tensioactif dans les échantillons tampon est de 3,4.10⁻² Mol/l. L'alcool gras libéré au cours de la réaction d'hydrolyse est extrait à l'éther diéthylique. Après chaque extraction les échantillons sont centrifugés (10 000 tours/min, 10 minutes) pour "casser" les émulsions formées. La solution éthérée est ensuite analysée par chromatographie en phase gazeuse sur colonne AT1 (polydiméthylsiloxane) apolaire en utilisant le n-dodécanol comme étalon interne. Les conditions mises au point sont les suivantes: injecteur à 320 °C, détecteur à 330 °C et gradient de température dans le four; 200 °C (3 min.), 30 °C/min (4 min.), 320 °C (5 min.). La figure unique représente les résultats obtenus.

### Exemple 5: Formulation d'un shampoing acide utilisant un mélange à base de mésylate de bétaïnate de 9-octadécényle

| | |
|---|---|
| Mélange obtenu dans l'exemple 2 | 32 |
| Diéthanolamide de coprah | 4 |
| Méthylparabène | 0,1 |
| Propylparabène | 0,1 |
| Rose pale du Maroc (rosa centifolia) | 0,5 |
| Eau déminéralisée | qsp 100 |
| Acide citrique | qsp pH 5 |

### Exemple 6: Formulation d'un shampoing acide utilisant un mélange à base de mésylate de bétaïnate d'octadécyle

| | |
|---|---|
| Mélange obtenu dans l'exemple 1, traitement C | 18,75 |
| Diéthanolamide de coprah | 4 |
| Méthylparabène | 0,1 |
| Propylparabène | 0,1 |
| Rose pale du Maroc (rosa centifolia) | 0,5 |
| Eau déminéralisée | qsp 100 |
| Acide citrique | qsp pH 5 |

## Revendications

1. Composition tensioactive comprenant:
au moins un composé de formule (1)
X⁻ (CH₃)₃N⁺-CH₂-CO-Z-R (1)
et au moins un composé choisi parmi ceux des formules (2), (3) et (4)
RZH (2)
XH (3)
X⁻ (CH₃)₃N⁺-CH₂-CO-OH (4)
X étant un radical sulfonate,
R étant un radical monovalent de formule C₂ₙH₂₍₂ₙ₋ₘ₎₊₁ contenant 2n atomes de carbone et m doubles liaisons, avec 9 ≤ n s 11, 0 ≤ m ≤ 3 si.n = 9 et 0 ≤ m ≤ 1 si n > 9, et
Z étant choisi parmi un atome d'oxygène et un groupe -NH-, un composé de formule XH étant combiné le cas échéant à au moins un composé de formule RNH₂ pour former au moins un composé de formule X⁻ RN⁺H₃.

2. Composition selon la revendication 1, comprenant pratiquement exclusivement des composés desdites formules (1), (2), (3), et (4).

3. Composition selon l'une des revendications 1 et 2, dans laquelle m = 0 et les composés constituant la composition sont les suivants, dans les pourcentages en poids indiqués:
| | |
|---|---|
| X⁻ (CH₃)₃N⁺-CH₂-CO-O-R | 50 ± 10 |
| ROH | 19 ± 10 |
| XH | 23 ± 10 |
| X⁻ (CH₃)₃N⁺-CH₂-CO-OH | 0 à 18. |

4. Composition selon l'une des revendications 1 et 2, dans laquelle m = 0 et les composés constituant la composition sont les suivants, dans les pourcentages en poids indiqués:
| | |
|---|---|
| X⁻ (CH₃)₃N-CH₂-CO-O-R | 72 ± 10 |
| ROH | 0 à 20 |
| XH | 0 à 18 |
| X⁻ (CH₃)₃N⁺-CH₂-CO-OH | 0 à 20. |

5. Composition selon l'une des revendications 1 et 2, dans laquelle m = 0 et les composés constituant la composition sont les suivants, dans les pourcentages en poids indiqués:
| | |
|---|---|
| X⁻ (CH₃)₃N⁺-CH₂-CO-O-R | 80 ± 10 |
| ROH | 20 ± 10. |

6. Composition selon l'une des revendications 1 et 2, dans laquelle m = 0 et les composés constituant la composition sont les suivants, dans les pourcentages en poids indiqués:
| | |
|---|---|
| X⁻ (CH₃)₃N⁺-CH₂-CO-O-R | 70 ± 10 |
| ROH | 26 ± 10 |
| XH | 0 à 14. |

7. Composition selon l'une des revendications 1 et 2, dans laquelle m > 0 et les composés constituant la composition sont les suivants, dans les pourcentages en poids indiqués:
| | |
|---|---|
| X⁻ (CH₃)₃N⁺-CH₂-CO-O-R | 48 ± 10 |
| ROH | 36 ± 10 |
| XH | 14 ± 10 |
| X⁻ (CH₃)₃N⁺-CH₂-CO-OH | 0 à 12. |

8. Composition selon l'une des revendications 1 et 2, dans laquelle m = 0 et les composés constituant la composition sont les suivants, dans les pourcentages en poids indiqués:
| | |
|---|---|
| X⁻ (CH₃)₃N⁺-CH₂-CO-NH-R | 58 ± 10 |
| X⁻ RN⁺H₃ | 35 ± 10 |
| X⁻ (CH₃)₃N⁺-CH₂-CO-OH | 0 à 20. |

9. Composition selon l'une des revendications 1 et 2, dans laquelle m > 0 et les composés constituant la composition sont les suivants, dans les pourcentages en poids indiqués:
| | |
|---|---|
| X⁻ (CH₃)₃N⁺-CH₂-CO-NH-R | 56 ± 10 |
| X⁻ RN⁺H₃ | 31 ± 10 |
| RNH₂ | 0 à 18 |
| X⁻ (CH₃)₃N⁺-CH₂-CO-OH | 0 à 15. |

10. Composition selon l'une des revendications précédentes, dans laquelle X est choisi parmi les radicaux méthane-sulfonate, paratoluènesulfonate et camphosulfonate.

11. Procédé de préparation d'une composition selon l'une aes revendications 1 et 2, dans lequel on fait réagir la glycine bétaïne avec un acide sulfonique et un composé de formule ROH en l'absence d'autres solvants, le rapport molaire de l'acide sulfonique à la glycine bétaïne étant compris entre 2 et 3 et le rapport molaire du composé ROH à la glycine bétaïne étant compris entre 1 et 1,5.

12. Procédé selon la revendication 11, dans lequel la réaction est conduite à une température comprise entre 130 et 140 °C pendant 6 à 8 heures.

13. Procédé selon l'une des revendications 11 et 12 dans lequel la réaction est conduite sous une préssion réduite, de préférence comprise entre 5000 et 10000 Pa.

14. Procédé selon l'une des revendications 11 à 13 dans lequel m = 0 et on traite le mélange réactionnel par un solvant organique propre à dissoudre préférentiellement les composés autres que X⁻ (CH₃)₃N⁺-CH₂-CO-O-R contenus dans celui-ci de manière à obtenir un précipité enrichi en X⁻ (CH₃)₃N⁺-CH₂-CO-O-R.

15. Procédé selon la revendication 14, dans lequel ledit solvant organique est choisi parmi l'éther diéthylique, l'éthanol et le n-butanol.

16. Procédé de préparation d'une composition selon l'une aes revendications 1 et 2, dans lequel on fait réagir la glycine bétaïne avec un acide sulfonique et le n-butanol en l'absence d'autres solvants, le rapport molaire de l'acide sulfonique à la glycine bétaïne étant compris entre 1 et 1,3 et le rapport molaire du n-butanol à la glycine bétaïne étant compris entre 2 et 4, pour former de l'eau et le sulfonate de l'ester n-butylique de glycine bétaïne, qu'on fait réagir, après avoir éliminé l'eau et le n-butanol, avec le composé de formule RNH₂, le rapport molaire de ce composé à la glycine bétaïne étant compris entre 1 et 1,2.

17. Procédé selon la revendication 16, dans lequel la réaction de la glycine bétaïne avec l'acide sulfonique et le n-butanol est conduite à une température comprise entre 130 et' 140 °C, au reflux du n-butanol, pendant 3 à 5 heures sous pression atmosphérique de manière à réaliser l'élimination azéotropique de l'eau.

18. Procédé selon l'une des revendications 16 et 17, dans lequel on ajoute, avant le composé de formule RNH₂, une base organique forte et encombrée, notamment la dibutylamine, le rapport molaire de ladite base à la glycine bétaïne étant compris entre 0,1 et 0,4.

19. Procédé selon l'une des revendications 16 à 18, dans lequel m = 0 et on traite le mélange réactionnel par un solvant organique propre à dissoudre préférentiellement le composé RNH₂ de manière à obtenir un précipité enrichi en X⁻ (CH₃)₃N⁺-CH₂-CO-NH-R.

20. Procédé selon la revendication 19, dans lequel ledit solvant organique est l'éther diéthylique.

21. Procédé selon l'une des revendications 11 à 20, dans lequel ledit acide sulfonique est choisi parmi l'acide méthanesulfonique, l'acide paratoluènesulfonique et l'acide camphosulfonique.

22. Cosmétique, en particulier savon liquide, bain moussant, gel douche ou shampoing et notamment shampoing acide, comprenant de 0,2 à 60 % et de préférence de 10 à 45 % en poids d'une composition selon l'une des revendications 1 à 10 et de 99,8 à 40% et de préférence de 90 à 55 % en poids d'excipients appropriés en cosmétologie.

23. Cosmétique selon la revendication 22, dans lequel les excipients sont choisis parmi des épaississants, des texturants, des agents de conditionnement, des adoucissants, des complexants, des parfums, des agents nacrants, des conservateurs, des acidifiants et de l'eau purifiée.

24. Cosmétique selon la revendication 23, dans lequel les excipients comprennent en tant que texturants des diéthanolamides d'acides gras, notamment le diéthanolamide de coprah, à une teneur ne dépassant pas 10 % en poids du cosmétique.

## Patentansprüche

1. Tensidzusammensetzung, mit:
wenigstens einem Bestandteil mit der Formel (1)
X⁻ (CH₃)₃N⁺-CH₂-CO-Z-R (1)
und wenigstens einem Bestandteil gewählt aus jenen der Formeln (2), (3) und (4)
RZH (2)
XH (3)
X⁻ (CH₃)₃N⁺-CH2-CO-OH (4)
wobei X ein Sulfonatradikal ist,
wobei R ein monovalentes Radikal der Formel C₂ₙH₂₍₂ₙ₋ₘ₎₊₁ ist, das 2n Kohlenstoffatome und m Doppelverbindungen enthält, mit 9 ≤ n ≤ 11,0 ≤ m ≤ 3, wenn n = 9 und 0 ≤ m ≤ 1, wenn n > 9, und
wobei Z gewählt wird aus einem Sauerstoffatom und einer Gruppe -NH-, wobei ein Bestandteil der Formel XH gegebenenfalls mit wenigstens einem Bestandteil der Formel RNH₂ kombiniert wird, um wenigstens einen Bestandteil der Formel X⁻ RN⁺H₃ zu bilden.

2. Zusammensetzung nach Anspruch 1, die praktisch ausschließlich Bestandteile der Formeln (1), (2), (3) und (4) ausweist.

3. Zusammensetzung nach Anspruch 1 oder 2, bei der m = 0 ist und die Bestandteile, die die Zusammensetzung bilden, die folgenden sind in den angegebenen Gewichtsprozenten:
| | |
|---|---|
| X⁻ (CH₃)₃N⁺-CH₂-CO-O-R | 50 ± 10 |
| ROH | 19 ± 10 |
| XH | 23 ± 10 |
| X⁻ (CH₃)₃N⁺-CH₂-CO-OH | 0 bis 18 |

4. Zusammensetzung nach Anspruch 1 oder 2, bei der m = 0 ist und die Bestandteile, die die Zusammensetzung bilden, die folgenden sind in den angegebenen Gewichtsprozenten:
| | |
|---|---|
| X⁻ (CH₃)₃N⁺-CH₂-CO-O-R | 72 ± 10 |
| ROH | 0 bis 20 |
| XH | 0 bis 18 |
| X⁻ (CH₃)₃N⁺-CH₂-CO-OH | 0 bis 20. |

5. Zusammensetzung nach Anspruch 1 oder 2, bei der m = 0 ist und die Bestandteile, die die Zusammensetzung bilden, die folgenden sind in den angegebenen Gewichtsprozenten:
| | |
|---|---|
| X⁻ (CH₃)₃N⁺-CH₂-CO-O-R | 80 ± 10 |
| ROH | 20 ± 10. |

6. Zusammensetzung nach Anspruch 1 oder 2, bei der m = 0 ist und die Bestandteile, die die Zusammensetzung bilden, die folgenden sind in den angegebenen Gewichtsprozenten:
| | |
|---|---|
| X⁻ (CH₃)₃N⁺-CH₂-CO-O-R | 70 ± 10 |
| ROH | 26 ± 10 |
| XH | 0 bis 14. |

7. Zusammensetzung nach Anspruch 1 oder 2, bei der m > 0 ist und die Bestandteile, die die Zusammensetzung bilden, die folgenden sind in den angegebenen Gewichtsprozenten:
| | |
|---|---|
| X⁻ (CH₃)₃N⁺-CH₂-CO-O-R | 48 ± 10 |
| ROH | 36 ± 10 |
| XH | 14 ± 10 |
| X⁻ (CH₃) ₃N⁺-CH₂-CO-OH | 0 bis 12. |

8. Zusammensetzung nach Anspruch 1 oder 2, bei der m = 0 ist und die Bestandteile, die die Zusammensetzung bilden, die folgenden sind in den angegebenen Gewichtsprozenten:
| | |
|---|---|
| X⁻ (CH₃)₃N⁺-CH₂-CO-NH-R | 58 ± 10 |
| X⁻ RN⁺H₃ | 35 ± 10 |
| X⁻ (CH₃)₃N⁺-CH₂-CO-OH | 0 bis 20. |

9. Zusammensetzung nach Anspruch 1 oder 2, bei der m > 0 ist und die Bestandteile, die die Zusammensetzung bilden, die folgenden sind in den angegebenen Gewichtsprozenten:
| | |
|---|---|
| X⁻ (CH₃)₃N⁺-CH₂-CO-NH-R | 56 ± 10 |
| X⁻ RN⁺H₃ | 31 ± 10 |
| RNH₂ | 0 bis 18 |
| X⁻ (CH₃)₃N⁺-CH₂-CO-OH | 0 bis 15. |

10. Zusammensetzung nach einem der vorhergehenden Ansprüche, in der X ausgewählt wird aus den Radikalen Methansulfonat, para-Toluolsulfonat und Camphersulfonat.

11. Verfahren zur Zubereitung einer Zusammensetzung nach Anspruch 1 oder 2, bei dem Glycinbetain mit einer Sulfonsäure und einer Zusammensetzung der Formel ROH bei Nichtvorhandensein von anderen Lösungsmitteln reagieren lässt, wobei das Molverhältnis der Sulfonsäure zum Glycinbetain zwischen 2 und 3 und das Molverhältnis der Zusammensetzung ROH zum Glycinbetain zwischen 1 und 1,5 liegt.

12. Verfahren nach Anspruch 1, bei dem die Reaktion bei einer Temperatur zwischen 130 und 140 °C während 6 bis 8 Stunden geführt wird.

13. Verfahren nach Anspruch 11 oder 12, bei dem die Reaktion unter einem verringerten Druck, vorzugsweise zwischen 5000 und 10000 Pa, geführt wird.

14. Verfahren nach einem der Ansprüche 11 bis 13, bei dem m = 0 ist und die Reaktionsmischung mit einem organischen Lösungsmittel behandelt wird, das in der Lage ist, vorzugsweise die Zusammensetzungen anders als X⁻ (CH₃)₃N⁺-CH₂-CO-O-R, die in dieser enthalten ist, aufzulösen, derart, dass eine an X⁻ (CH₃)₃N⁺-CH₂-CO-O-R angereicherte Ausfällung erhalten wird.

15. Verfahren nach Anspruch 14, bei dem das organische Lösungsmittel ausgewählt wird aus dem Diethylether, dem Ethanol und dem n-Butanol.

16. Verfahren zur Zubereitung einer Zusammensetzung nach Anspruch 1 oder 2, bei dem man das Glycinbetain mit einer Sulfonsäure und dem n-Butanol bei Abwesenheit von anderen Säuren reagieren lässt, wobei das Molverhältnis der Sulfonsäure zum Glycinbetain zwischen 1 und 1,3 liegt und das Molverhältnis des n-Butanol zum Glycinbetain zwischen 2 und 4 liegt, um Wasser und das Sulfonat des n-Butylesters des Glycinbetains zu bilden, das man, nachdem das Wasser und das n-Butanol entfernt wurden, mit der Zusammensetzung der Formel RNH₂ reagieren lässt, wobei das Molverhältnis dieser Zusammensetzung zum Glycinbetain zwischen 1 und 1,2 liegt.

17. Verfahren nach Anspruch 16, bei dem die Reaktion des Glycinbetain mit der Sulfonsäure und dem n-Butanol bei einer Temperatur geführt wird, die zwischen 130 und 140 °C liegt, bei Rücklauf von n-Butanol, während 3 bis 5 Stunden unter Umgebungsdruck, derart, dass ein azeotropes Entfernen von Wasser verwirklicht wird.

18. Verfahren nach Anspruch 16 und 17, bei dem, vor der Zusammensetzung nach der Formel RNH₂, eine organische starke und störende Grundlage, insbesondere Dibutyl-amin, hinzugeführt wird, wobei das Molverhältnis der Grundlage zum Glycinbetain zwischen 0,1 und 0,4 liegt.

19. Verfahren nach einem der Ansprüche 16 bis 18, bei dem m = 0 ist und die Reaktionsmischung durch eine organische Lösung behandelt wird, die in der Lage ist, vorzugsweise die Zusammensetzung RNH₂ derart aufzulösen, dass eine Ausfällung angereichert an X⁻ (CH₃) ₃N⁺-CH₂-CO-NH-R erhalten wird.

20. Verfahren nach Anspruch 19, bei dem das organische Lösungsmittel Diethylether ist.

21. Verfahren nach einem der Ansprüche 11 bis 20, bei dem die Sulfonsäure ausgewählt wird aus der Methansulfonsäure, der p-Toluolsulfonsäure und der Camphersulfonsäure.

22. Kosmetisches Mittel, insbesondere Flüssigseife, Schaumbad, Duschgel oder Shampoo und insbesondere Saures Shampoo, das 0,2 bis 60 Gewichts-% und vorzugsweise 10 bis 45 Gewichts-% einer Zusammensetzung nach einem der Ansprüche 1 bis 10 und 99,8 bis 40 Gewichts-% und vorzugsweise 90 bis 55 Gewichts-% Hilfsstoffe aufweist, die in der Kosmetik geeignet sind.

23. Kosmetisches Mittel nach Anspruch 22, bei dem die Hilfsstoffe ausgewählt werden aus Verdickungsmitteln, Texturierungsmitteln, Konditionierungsmitteln, Komplexierungsmitteln, Parfums, Perlglanzmitteln, Konservierungsmitteln, säurebildenden Mitteln und gereinigtem Wasser.

24. Kosmetisches Mittel nach Anspruch 23, bei dem die Hilfsstoffe als Texturierungsmittel Diäthanolamide ungesättigter Fettsäuren, insbesondere das Diäthanolamid von Kopra aufweisen, mit einem Gehalt, der 10 Gewichts-% des kosmetischen Mittels nicht übersteigt.

## Claims

1. Surfactant composition comprising:
at least one compound of formula (1)
X⁻ (CH₃)₃N⁺-CH₂-CO-Z-R (1)
and at least one compound selected from those with formulae (2), (3) and (4)
RZH (2)
XH (3)
X⁻(CH₃)₃N⁺-CH₂-CO-OH (4)
X being a sulphonate radical,
R being a monovalent radical of formula C₂ₙH₂₍₂ₙ₋ₘ₎₊₁ containing 2n atoms of carbon and m double bonds, with 9 ≤ n ≤ 11,0 ≤ m ≤ 3 if n = 9 and 0 ≤ m ≤ 1 if n > 9, and
Z being selected from an atom of oxygen and a -NH- group, a compound of formula XH being combined if necessary with at least one compound of formula RNH₂ in order to form at least one compound of formula X⁻RN⁺H₃.

2. Composition according to claim 1, comprising virtually exclusively compounds of the said formulae (1), (2), (3) and (4).

3. Composition according to either of claims 1 or 2, wherein m = 0 and the compounds forming the composition are as follows, in the percentages by weight indicated:
| | |
|---|---|
| X⁻ (CH₃)₃N⁺-CH₂-CO-O-R | 50 ± 10 |
| ROH | 19 ± 10 |
| XH | 23 ± 10 |
| X⁻ (CH₃)₃N⁺-CH₂-CO-OH | 0 to 18. |

4. Composition according to either of claims 1 or 2, wherein m = 0 and the compounds forming the composition are the following, in the percentages by weight indicated:
| | |
|---|---|
| X⁻ (CH₃)₃N⁺-CH₂-CO-O-R | 72 ± 10 |
| ROH | 0 to 20 |
| XH | 0 to 18 |
| X⁻ (CH₃)₃N⁺-CH₂-CO-OH | 0 to 20. |

5. Composition according to either of claims 1 or 2, wherein m = 0 and the compounds forming the composition are the following, in the percentages by weight indicated:
| | |
|---|---|
| X⁻ (CH₃)₃N⁺-CH₂-CO-O-R | 80 ± 10 |
| ROH | 20 ± 10. |

6. Composition according to either of claims 1 or 2, wherein m = 0 and the compounds forming the composition are the following, in the percentages by weight indicated:
| | |
|---|---|
| X⁻ (CH₃)₃N⁺-CH₂-CO-O-R | 70 ± 10 |
| ROH | 26 ± 10 |
| XH | 0 to 14. |

7. Composition according to either of claims 1 or 2, wherein m > 0 and the compounds forming the composition are the following, in the percentages by weight indicated:
| | |
|---|---|
| X⁻ (CH₃)₃N⁺-CH₂-CO-O-R | 48 ± 10 |
| ROH | 36 ± 10 |
| XH | 14 ± 10 |
| X⁻ (CH₃)₃N⁺-CH₂-CO-0H | 0 to 12. |

8. Composition according to either of claims 1 or 2, wherein m = 0 and the compounds forming the composition are the following, in the percentages by weight indicated:
| | |
|---|---|
| X⁻ (CH₃)₃N⁺-CH₂-CO-NH-R | 58 ± 10 |
| X⁻ RN⁺H₃ | 35 ± 10 |
| X⁻ (CH₃)₃N⁺-CH₂-CO-OH | 0 to 20. |

9. Composition according to either of claims 1 or 2, wherein m > 0 and the compounds forming the composition are the following, in the percentages by weight indicated:
| | |
|---|---|
| X⁻ (CH₃)₃N⁺-CH₂-CO-NH-R | 56 ± 10 |
| X⁻ RN⁺H₃ | 31 ± 10 |
| RNH₂ | 0 to 18 |
| X⁻ (CH₃)₃N⁺-CH₂-CO-OH | 0 to 15. |

10. Composition according to one of the preceding claims, wherein X is selected from the radicals methane sulphonate, paratoluene sulphonate and camphosulphonate.

11. Method of preparing a composition according to either of claims 1 or 2, wherein betaine glycine is reacted with a sulphonic acid and a compound of the formula ROH in the absence of other solvents, the molar ratio of sulphonic acid to betaine glycine being comprised between 2 and 3 and the molar ratio of the compound ROH to the betaine glycine being between 1 and 1.5.

12. Method according to claim 11, wherein the reaction is carried out at a temperature of between 130 and 140°C for 6 to 8 hours.

13. Method according to either of claims 11 or 12, wherein the reaction is carried out under reduced pressure, preferably of between 5,000 and 10,000 Pa.

14. Method according to one of claims 11 to 13, wherein m = 0 and the reactional mixture is treated with an organic solvent capable of dissolving preferably the compounds other than the X⁻ (CH₃)₃N⁺-CH₂-CO-O-R contained therein so as to obtain a precipitate enriched in X- (CH₃)₃N⁺-CH₂-CO-O-R.

15. Method according to claim 14, wherein the organic solvent is selected from diethyl ether, ethanol and n-butanol.

16. Method of preparing a composition according to either of claims 1 or 2, wherein betaine glycine is reacted with a sulphonic acid and n-butanol in the absence of other solvents, the molar ratio of the sulphonic acid to betaine glycine being between 1 and 1.3 and the molar ration of the n-butanol to the betaine glycine being between 2 and 4, in order to form water and the sulphonate of the n-butylic ester of betaine glycine, which is reacted, after elimination of the water and n-butanol, with the compound having the formula RNH₂, the molar ratio of this compound to betaine glycine being between 1 and 1.2.

17. Method according to claim 16, wherein the reaction of the betaine glycine with the sulphonic acid and the n-butanol is carried out at a temperature of between 130 and 140°C, at the reflux of n-butanol, for 3 to 5 hours under atmospheric pressure so as to effect azeotropic elimination of water.

18. Method according to one of claims 16 or 17, wherein, before the compound of formula RNH₂, is added a strong, encumbered organic base, in particular dibutylamine, the molar ratio of the base to the betaine glycine being between 0.1 and 0.4.

19. Method according to either of claims 16 to 18, wherein m = 0 and the reactional mixture is treated with an organic solvent capable of dissolving preferably the compound RNH₂ so as to obtain a precipitate enriched in X⁻ (CH₃)₃N⁺-CH₂-CO-NH-R.

20. Method according to claim 19, wherein the organic solvent is diethyl ether.

21. Method according to one of claims 11 to 20, wherein the sulphonic acid is selected from methanesulphonic acid, paratoluenesulphonic acid and camphosulphonic acid.

22. Cosmetic, in particular liquid soap, foam bath, shower gel or shampoo and in particular acid shampoo comprising 0.2 to 60% and preferably 10 to 45% by weight of a composition according to one of claims 1 to 10 and 99.8 to 40% and preferably 90 to 55% by weight excipients appropriate for cosmetology.

23. Cosmetic according to claim 22, wherein the excipients are selected from thickeners, texturisers, conditioning agents, softeners, complexing agents, perfumes, pearlising agents, preservatives, acidifiers and purified water.

24. Cosmetic according to claim 23, wherein the excipients comprise as texturisers diethanolamides of fatty acids, in particular diethanolamide of copra, with a content not exceeding 10% by weight of the cosmetic.
